# EUROPEAN PATENT SPECIFICATION

(11) **EP 4 728 273 B1**
(45) Date of publication and mention of the grant of the patent: **21.10.2026**
(21) Application number: 24722154.2
(22) Date of filing: 23.04.2024
(51) Int. Cl.: G01N 33/18, G01N 21/64

(54) **DEVICE FOR MONITORING TOXIC SUBSTANCES IN WATER AND SYSTEM COMPRISING THE SAME**
VORRICHTUNG ZUR ÜBERWACHUNG VON TOXISCHEN SUBSTANZEN IN WASSER UND SYSTEM DAMIT
DISPOSITIF DE SURVEILLANCE DE SUBSTANCES TOXIQUES DANS L'EAU ET SYSTÈME LE COMPRENANT

(30) Priority: 13.06.2023 ES 202330495
(43) Date of publication of application: 22.04.2026
(73) Proprietor: AECOM SPAIN DCS, S.L., 28014 Madrid (ES)
(72) Inventor: CARAFA, Roberta, 08010 Barcelona (ES); CANUTI, Elisabetta, 21027 Ispra (VA) (IT); GALPAROLI, Marco, 21027 Ispra (VA) (IT)
(74) Representative: Clarke, Modet y Cía., S.L.
(86) International application number: PCT/EP2024/061118
(87) International publication number: WO 2024/256070

(56) References cited:
- WO-A1-2021/205051
- DE-C1- 19 647 201

## Description

### Technical field of the Invention

The invention pertains to the field of water quality and safety monitoring. More specifically, it relates to systems and methods for the early detection of micro-pollutants in surface waters.

### Background of the Invention

Traditionally, the assessment of pollutants in water depends on water sampling and the extraction and analysis of pollutants by chromatography and mass spectrometry, but chemical analysis is expensive and time consuming. Specialized technicians are required for sampling and analysis. Compounds can only be detected in previously calibrated detector instruments. Due to the scarce information on the toxic effects of the mixtures, there are difficulties in the evaluation of the impact in water ecosystems and humans.

In situ probes are currently available for aquatic ecosystems capable of transmitting signals and alerts, although they only measure hydraulic parameters (e.g., water level, flow) and inorganic chemical components (e.g., oxygen, pH, suspended solids, etc.). In general, these probes have several disadvantages: they are for specific substances and/or are intended for laboratory use and/or are expensive and/or have demanding maintenance and/or interpretation of the results is difficult.

Some analysis techniques use biofilms (also called bacterial tapestry or microbial mat) as a bio-indicator of water quality and safety. By biofilm is meant a film formed by microalgae, diatoms and/or bacteria. Generally, these analysis techniques have to be done in situ and provide unreliable results. They have high variability and are subject to interpretation, making them often impractical. On the one hand, biofilm-based indicators are influenced by a multitude of variables not necessarily related to water pollution, such as light intensity, suspended solids, nutrients, nitrates, nitrites, phosphorus, temperature, speed of water flow, etc. that affect the validity of the results.

On the other hand, the biofilm can adapt to water conditions and develop specific resistances that affect its sensitivity. Accordingly, the results of biofilm-based indicators may be biased.

WO2021/205051 discloses a device for monitoring toxic substances in water, with a monitoring chamber and a reference chamber, each of these chambers comprising an inlet and an outlet for the water flow, a biofilm and a fluorimeter. A purifying filter is arranged before the reference chamber. In both chambers the biofilm can receive outside light. A data acquisition unit acquires the fluorescence measured by the fluorometers.

### Summary of the invention

In view of the limitations observed in the prior art, there has been a need to improve the diagnosis of water.

In addition, rapid detection would be desirable, especially in places of interest. For example, at the exit of wastewater treatment Plants (WWTPs) or at points for drinking water supply. Also at the entrance of a WWTP it is especially useful because the peaks of pesticides affect the biological processes of abatement of organic substance in the WWTP. Thus, an early warning can provide important information for WWTP managers, so that they can act quickly.

The present invention is devised to address these and other problems. A device with the technical features of the independent claim is proposed. Particular and advantageous embodiments are defined in the dependent claims.

In general, a device is proposed for monitoring toxic substances in water that includes a body with two chambers arranged in series following the direction of water flow. Both chambers are separated by a purifying filter. The material of the purifying filter can be chosen according to the type of pollutants that are expected to be found. The device includes an inlet so that, in use, the water flow can first pass through a first chamber where it is monitored. The device includes a second chamber, located after the first chamber and after the purifying filter, and an outlet for the water flow. The device may be symmetrical, and according to the flow direction, the first chamber is determined to be a monitoring chamber and the second is a reference chamber for comparison with filtered water. The device incorporates in each chamber a housing module for housing at least one biofilm, and a fluorometer for measuring the fluorescence of the biofilm. In use, the housing module should receive exterior light for the biofilm. The device further incorporates a data acquisition unit for collecting the fluorescence measurement of the biofilm in each chamber. Thus, a local comparison can be established between a biofilm with water purified of toxic substances and another biofilm with untreated water and, thereby, discriminate changes due to pollutants. Preferably, the acquisition unit is positioned externally, outside the chambers. Optionally, in embodiments with greater electronic implementation, the data acquisition unit may incorporate capability to process information and communicate with other devices.

The device may optionally incorporate other measuring elements such as passive samplers for organic pollutants and metals, also phytoplankton capture filters. By extracting the sample contained in the passive samplers and analyzing it, which can be analyzed a posteriori in the laboratory, the use of these passive samplers in the device provides supplementary information. Such supplementary information serves as confirmation that the changes in the biofilm are actually due to pollutants. It also serves to recognize if the operation of the filters is correct. Similarly with the phytoplankton capture filter.

This specificity allows us to rule out changes in the biofilm caused by other factors. For example, there are variations in climatic conditions that are unrelated to pollution but that cause changes in the biofilm and that, therefore, could generate false positives of pollution.

Additionally, another advantage of the device is that the water flow through the first chamber is the same as that through the second chamber. This avoids that there is a difference with respect to the water flow in each chamber where the measuring elements are and that this factor can affect the result.

Embodiments of the device are considered to be connected to a hydraulic pump that ensures a certain water flow through the chambers. In such embodiments the device is located on the surface, out of the water. By means of two taps, one placed at the inlet and one at the outlet of the device: both can regulate the flow, or to close the chamber in case of need to transport the biofilm. Other embodiments may operate with the body of the device submerged, in a location that ensures a certain continuous water flow through the chambers of the device. For example, some embodiments of the device may be coupled with an adapted drain conduction that ensures a given water flow through the chambers.

The device may be coupled with or optionally include a data communication unit. The data communication unit can send and receive information from other remote systems in charge of reviewing the information and acting accordingly. For example, you can take actions such as generating alarms due to the type of pollution, maintenance notices, etc. It is also compatible with other tools and accessories that can be incorporated to adapt to specific requirements.

The invention also relates to a system that incorporates one or more monitoring devices in different locations and/or capable of detecting different toxic substances in water. In addition, the system includes a remote computer in communication with each monitoring device. The remote computer is programmed to compare the measurements acquired in both chambers of each device and further to send an alarm to one or more surveillance terminals based on the result of the comparison. These terminals are electronic devices such as mobile phones, tablets, computers, etc. In embodiments in which various sensors are included in one or more monitoring devices the computer may be further programmed to control them.

### Brief description of the drawings

To complement the description that is being made and in order to help a better understanding of the characteristics of the invention, a set of drawings is attached as an integral part of said description, in which, with an illustrative and non-limiting nature, the following has been represented:
Fig. 1.- Schematic block diagram according to an embodiment of the device.
Fig. 2.- Schematic exploded view of an embodiment of the device.

### Numerical references

1 Monitoring chamber.
2 Reference chamber.
12 Purification chamber.
3 Passive sampler for organic pollutants.
4 Passive sampler for inorganic pollutants.
5 Biofilm.
6 Fluorometer.
7 Phytoplankton capture filter.
8 Purifying filter.
11Filter mesh.
13 Water tightness gasket.
9 Pre-filter.
10 Housing module.
14 Outlet port for wiring.
16 Underwater cable.
17 Joint piece.
18 Data acquisition unit.
19 Surface equipment.
20 Cloud server.
21 Remote computer.
22 Surveillance terminal.
23 Data communication unit.
24 Processor.
26 Renewable energy production system.
27 Battery.
28 Solar panel.
30 Toxic substance monitoring device.
31 Hydraulic pump.
33 Conduction pipe.
41 Connector parts
42 Tap

### Detailed description of the invention

For a more complete understanding of the invention, different aspects of particular embodiments are described with reference to the above figures.

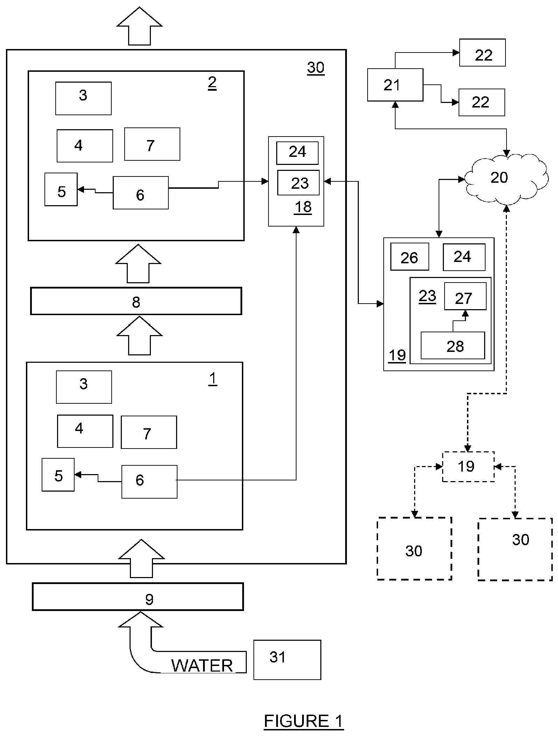

In **FIG. 1** is schematically shown a block diagram according to an embodiment, the monitoring device **30** allows, in a continuous manner, a prompt detection of the presence of pollution in the water. The device **30** uses biofilm **5.** Biofilm **5** is a very sensitive living community and serves as the primary detection element. The device **30** has a configuration that avoids interpretation errors generated by external factors not related to the presence of pollutants to be detected.

In operation, the device **30** detects changes by fluorescence measurements of biofilm **5** made in two different spaces with water from the aquatic environment to be monitored. To do this, each space has at least one fluorometer **6** (it can include several for reliability and redundancy). On a first space, changes in the environment due to the presence of toxic substances are monitored. A second space serves as a local reference with which to have conditions in the environment without toxic substances. Both spaces are connected in series, one following the other forming a body. To create these two spaces in contact with the aquatic environment, chambers 1, 2 are designed. Among other concrete properties, both chambers 1, 2 must allow the development of the biofilm 5. In case of pollution, one of them, the reference chamber 2 must keep the biofilm 5 in water purified of pollutants, in order to identify the change suffered by the other biofilm 5 that is in the monitoring chamber 1 exposed to pollutants that exist in the aquatic environment. Since both are in series, the water flow must first pass through the monitoring chamber 1, then through a purifying filter 8 and then through the reference chamber 2. One chamber is separated from the other chamber by inserting the purifying filter 8 to prevent the passage of pollutants.

Depending on the conditions of the water to be monitored, it may be necessary to pre-filter the water entering the monitoring chamber 1 to prevent the lifetime of the measuring elements and/or purification filter 8 from being too short. This pre-filtering can be performed with a pre-filter 9 that allows the passage to particles with a size smaller than a certain one, preferably smaller than 80 microns. In some embodiments, the pre-filter 9 is associated with a hydraulic pump 31 in charge of introducing the water into the device 10. In other embodiments, the device 30 can be immersed or installed in a drain conduction so as to ensure a continuous water flow through the chambers 1, 2.

In the reference chamber 2, the incoming water is purified of pollutants, but maintains other characteristics (temperature, pH, nutrients, etc.). The measurements of the biofilm 5 of each chamber 1, 2 are compared, of which a remote computer 21 is preferably in charge, although it is also possible that a surface equipment 19 is in charge in some embodiments. Based on this comparison, if there is a significant difference, a warning signal can be generated. With this warning signal, an alert can in turn be issued to be displayed on one or more surveillance terminals 22. The warning signal can be transmitted with a communications unit 23 easily using wireless technology (such as 3G, 4G, 5G, or WiFi telephone network).

The monitoring device 30 allows to verify the quality of the water on a continuous basis. The design also supports the use of passive samplers to detect contamination by pesticides, pharmaceutical compounds and heavy metals, among others. To do this, it can integrate one or more passive samplers for inorganic pollutants 4 (DGT) and one or more passive samplers for organic pollutants 3 (POCIS). These passive samplers 3, 4 can be used as a backup for chemical analysis and identification of pollutants. It can also include a phytoplankton capture filter 7.

The type of fluorometer 6 employed is preferably Pulse Amplitude Modulated (PAM) for rapid assessment of changes in autotrophic biofilm functional indicators related to photosynthetic yield.

To establish if the differences in measurement of the fluorometer 6 of the reference chamber 2 and the fluorometer 6 of the monitoring chamber 1 are significant, they are calibrated in the field and in the laboratory.

It should be considered that biofilm 5 is usually grown in clean water conditions. Biofilm 5 reacts to exposure to pollutants with changes in functional parameters (e.g., efficiency of photosynthesis, basal fluorescence, etc.). These changes are detectable by the fluorometer 6. Advantageously, the presence of a local reference prevents false positives. Often external factors unrelated to toxicity can affect the values measured in the biofilm. For example, these parameters are influenced by temperature, turbidity, nutrients, flow rate. Previously, adequate calibration is performed in the laboratory.

Generally, calibration is carried out through previous toxicity tests on the biofilm. The variation in photosynthetic efficiency is measured with the fluorometer, with exposure to various known concentrations of the pollutants (or mixtures of pollutants) targeted for field monitoring. These tests make it possible to verify the sensitivity of the biofilm. Other components can also be previously verified, such as the efficiency of the filters with laboratory tests, for example, by recirculating water with known pollutant concentration in the device and measuring the reduction of pollutant concentration in the water over time. With the measurements of the fluorometer, various parameters can be obtained, among which we can mainly mention:
- Yll, *photosynthetic yield* ranging from 0 to 100% theoretically, and with normal values for a biofilm in good condition between 60% and 70%;
- Basal fluorescence: represents an indirect measurement of biomass and can rise or grow or remain constant during exposure, (the range depends on the calibration of the sensor that is done prior to installation);
- Y(NPQ), *yield of regulated non-photochemical fluorescence quenching* that represents the energy that cells emit in the form of heat, is a protective mechanism and is an indicator of stress;
- And(NO), *yield of non-regulated non-photochemical fluorescence quenching* this parameter indicates a dysfunction of the photosynthesis and/or protection mechanisms.

In fact: YII+Y(NPQ)+Y(NO)=100%, if the cells lower their energy by photosynthesis, the energy spent on heat or used, for example, for detoxification will grow. Laboratory tests indicate the relationship between effect and toxic mixture. The local reference also allows us to appreciate small variations that would be confused with effects due to nontoxic factors.

A data acquisition unit 18 (which may be submersible in some embodiments) may be coupled to the body of the monitoring device 30, preferably in a wired manner, to a surface equipment 19, located outside the water and powered by a power production system 26, for example, of renewable energy, for example, a solar panel 28 that powers a battery 27 for greater autonomy. In said surface equipment 19, a communication unit 23 allows the possibility of sending, preferably wirelessly, the information acquired from the monitoring device 30 for processing, for example, to a remote computer 21 directly or through a cloud server 20. This remote computer 21 may, among other actions, be responsible for issuing an alert based on the data received, storing and analyzing the information from one or more distributed monitoring devices 30, etc. Different types of alerts may, for example, be established according to the estimated degree of toxicity. In some embodiments, as shown in dotted line in FIG. 1, a same surface equipment 19 may be associated with several monitoring devices 30 and which receive the acquired information identifying its origin. Grouping several devices 30 may be interesting in situations where it is possible to locate them next to the surface equipment 19.

In some electronically more complex embodiments, the monitoring device 30 itself may be provided with a communications unit 23 and have some autonomous processing capacity by a processor **24** associated with the data acquisition unit **18.** For example, it may be programmed to analyze information from one or more electronic sensors and generate a message with data in response. This message may additionally be sent by said communications unit **23.** It may also manage information received by the communications unit **23** such as, for example, a control instruction.

A network may be deployed with several monitoring devices **30** and surface equipment **19** either associated with a single monitoring device **30** or with several (dotted line). A system for monitoring different substances and/or monitoring different locations in a distributed manner can be achieved.

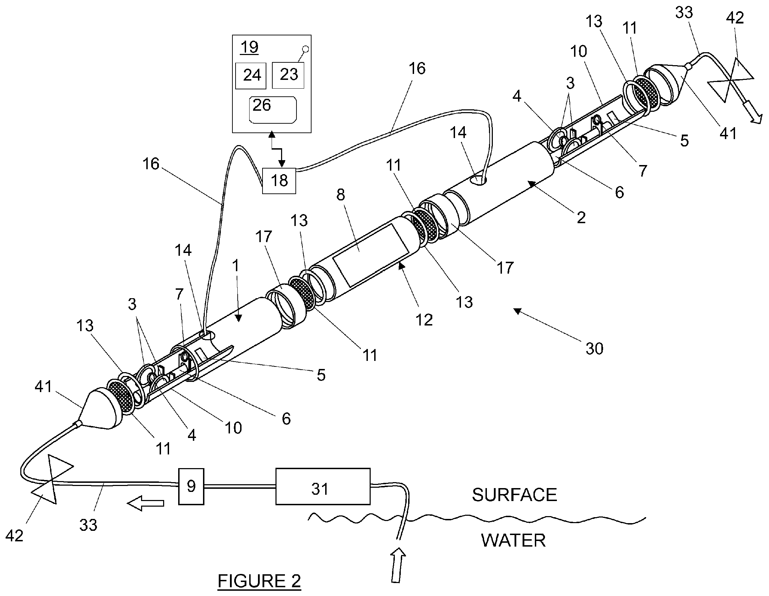

In **FIG. 2** several details and constructive aspects of a particular embodiment of the monitoring device **30** are represented. The device has a main body where there are three differentiated chambers, a monitoring chamber **1** and a reference chamber **2** transparent or translucent for the growth of the biofilm **5.** A purifying filter 8 is installed between the two. In this embodiment, a third purification chamber **12** is used to house said filter **8.** The purifying filter **8** may include activated carbon. The chambers **1, 2, 12** are tubular although they accept other geometries, it is enough to ensure an adequate water flow through the purifying filter **8.** For example, one possibility is to design the chambers in the shape of a spiral. Preferably, pipes are attached to each other by threading. A joint piece **17** is additionally employed to facilitate assembly.

Optionally, for example, in embodiments such as the one illustrated in FIG.2 where a hydraulic pump **31** is employed: at each end of the chambers **1, 2** funnel-shaped connector pieces **41** are installed, with a cylindrical part to couple with a conduction pipe **33** and with a conical part to avoid turbulence in the water flow. One of the conductions **33** attached to a hydraulic pump **31** may preferably be in position upstream of the device **30** as illustrated and taking water from the aquatic environment to be monitored. Prior to entering the device **30,** the water flow may be passed through a pre-filter **9** to prevent the introduction of particles of undesired size that saturate filters or cause a malfunction of samplers **3, 4, 7** or sensors **6.** There are two taps **42** in the conductions **33,** a first one is placed at the inlet to regulate the flow entering the device **30,** and a second one is placed at the outlet of the device **30** is employed generally open. Both are closed to allow transport of the device **30** from one site to another with the biofilm inside. The outlet tap may also regulate the flow through the device **30.** Mention that the elements before the chambers: the conduction pipe **33,** the pre-filter **9,** etc., must be of inert material (for example, Teflon or stainless steel). In contrast, the outlet conduction pipe **33** is not; for example, it may be made of plastic material.

Preferably, the body of the device **30** is symmetrical, i.e. the chambers **1, 2** are equal, and it is the direction of the water flow that determines whether it is the reference chamber **2** or the monitoring chamber **1.** This facilitates the assembly of the device and installation at its destination.

In other embodiments, the device **30** is submersible and can be installed in a drain conduction or evacuation pipeline so that, in use, the force of the same water stream transports the possible pollutants to the monitoring chamber for detection, allows the monitoring chamber **1** to purify the water and follow its passage through the reference chamber **2** to finally exit. In these embodiments, the connector parts **41** and the pump **31** would be dispensed with if the force of the current allows the water flow to be continuous in the device **30** (the water inside does not stagnate).

In the embodiment shown, several filter meshes **11** are seen. A filter mesh **11** is coupled on the one hand to the connector piece **41** and on the other hand it is coupled to a chamber **1, 2.** With a water tightness gasket **13** a good seal is ensured (the ring can be a Teflon gasket). Also included at both ends of the purification chamber **12** is a filter mesh **11** that prevents the accidental exit of elements of the purifying filter **8** but does not impede the water flow (coarser mesh, for example, 2 mm). For example, granular activated carbon having less opposition to the water flow may be used in the purification chamber **12** as the purifying filter 8. The water must arrive clean and without material from the purifying filter **8** itself to the biofilm **5** and/or to the fluorometer **6** and/or to the phytoplankton collection filter **7** and/or to the samplers **3, 4.**

The purifying filter **8** does not contain substances harmful to the biofilm **5,** for example, a biological filter with bacteria would be inappropriate since it could alter the biofilm. It must allow the water flow, for example, in case of presence of suspended material to prolong the life of the purifying filter **8** it is better to pre-filter the water previously with one or more pre-filters **9** and a hydraulic pump **31** is necessary to ensure the water flow. An advantage of the activated carbon in the purifying filter **8** is that it is reactivatable with heat and also reusable. Whether with activated carbon or a material of similar characteristics, the water entering the reference chamber **2** is purified. Polar organic pollutants are retained, generally with octanol-water partition coefficient, KOW≤3, and heavy metals.

Note: The octanol-water partition coefficient of a substance, also called the partition coefficient (POW), is the coefficient or ratio between the concentrations of that substance in a biphasic mixture formed by two immiscible solvents at equilibrium: n-octanol and water. This coefficient therefore measures the differential solubility of a solute in these two solvents. N-octanol has been chosen for being an organic compound that simulates either the lipid material of biota, or in organic particles and sediments. This coefficient gives an idea of the hydrophobic character of a substance or the affinity towards lipids of a substance dissolved in water.

The chambers **1, 2, 12** can be manufactured with cylindrically shaped pipes in methacrylate (PMMA), sometimes commonly referred to as Plexiglass (brand under which it is marketed) for example, with a thickness of about 10 mm, inner diameter of 120 mm, and a length of 400 mm for the purification chamber **12** and for the chambers **1, 2.** They are designed with a capacity of several liters to ensure that the biofilm **5** has a water flow, light and specific nutrients to survive and to ensure water purification. Among other considerations, it must allow the passage of light, be sufficiently resistant to withstand the working conditions, and be inert with respect to the substances to be analyzed. For example, PET is not suitable because it can adsorb pollutants. Preferably, it should be a low-cost material, resistant to acids and diluted solvents to make it easy to clean. The dimensions of the purifying filter 8 must be chosen to ensure a certain contact time between water and activated carbon. The above dimensions are only a demonstrative example.

In some less preferred embodiments screws or rivets may be used and it should be kept in mind that they must be made of inert materials. For these parts, stainless steel or polytetrafluoroethylene (PTFE) also known as Teflon are suitable materials. For this reason, in the illustrated embodiment, the chambers are made from pipes that are easily screwed into each other, which saves screwing numerous screws or placing rivets and reduces the complexity of the assembly.

The hydrodynamic design of the two chambers **1, 2** must ensure adequate retention of pollutants. For example, a cylindrical shape ensures a uniform distribution of the water flow passing through the filters. Also the characteristics of filters must take into account the contact time between water and activated carbon and be sufficient to allow purification. Since biofilm **5** is a living microbial community with regeneration capacity, it requires little maintenance.

It is advisable to improve the efficiency of the device, that the biofilm 5 is grown in a clean spot or in laboratory and then transferred to the device. In this way, it is facilitated that the most sensitive species are present.

The design of the device **30** should allow relative isolation of the biofilm within the reference chamber 2 and the monitoring chamber **1,** which minimizes the colonization of resistant species. A balance is sought that slows colonization by resistant species without completely isolating the biofilm **5** to maintain water flow. The extracts from the passive samplers **3, 4, 7** can be analyzed for a better determination of the quality and characteristics of the water. In this way it is also possible to ensure not only a better identification of contaminants but also the proper functioning of the purifying filter **8.**

Versatile, the properties of the filter **8,** and pre-filter **9** can be chosen to specifically adapt to the local conditions of the water to be analyzed. Typically, the lifespan of a filter ranges from two weeks to several months, depending on the quality of the water.

Integrated passive samplers **3, 4** that are capable of accumulating organic compounds such as pesticides, pharmaceuticals and heavy metals. These compounds can be recovered to be analyzed in the laboratory, for example, with chromatography and mass spectrophotometry techniques known in the prior art. A significant change detected in the biofilm **5** can be detected. With this information, an average expert can design an appropriate mode of action against a specific pollution episode. Even adsorbents can be extracted and analyzed, providing valuable information, for example, to identify potential target compounds responsible for toxic effetsand the expected toxic effects on water from what is recorded in the biofilm.

Without limitation, the information provided for the various embodiments could be combined into additional embodiments within the scope of the invention determined by the following claims.

## Claims

1. A device (30) for monitoring toxic substances in water, **characterized in that** it comprises:
- a first monitoring chamber (1) with an inlet and an outlet for the water flow, comprising a housing module (10) configured to house at least one biofilm (5) and a fluorometer (6), wherein the fluorometer is configured to measure the fluorescence of the biofilm (5);
- a second reference chamber (2) with an inlet and an outlet for the water flow, comprising a housing module (10) configured to house at least one biofilm (5) and a fluorometer (6), wherein the fluorometer is configured to measure the fluorescence of the biofilm (5);
- a purifying filter (8) located between the first chamber (1) and the second chamber (2) so that the same water flow runs through the first chamber (1) and the second chamber (2) after passing through the purifying filter (8);
wherein both chambers (1,2) are configured to allow, in use, the biofilm (5) to receive outside light;
wherein the device (30) further comprises a data acquisition unit (18) configured to acquire the fluorescence measurement from the fluorometer (6) of each chamber (1,2).

2. Device (30) according to claim 1, further comprising a passive sampler for organic pollutants (3).

3. The device (30) according to claim 1 or 2, further comprising a passive sampler for inorganic pollutants (4).

4. Device (30) according to any one of claims 1 to 3, further comprising a phytoplankton capture filter (7).

5. Device (30) according to any one of the preceding claims, comprising a filter mesh (11) at the inlet and outlet of the chamber (1,2) for the water flow, wherein the filter mesh (11) is inert.

6. Device (30) according to claim 5, wherein the filter mesh (11) is 316 stainless steel.

7. Device (30) according to any one of the preceding claims, wherein the purifying filter (8) comprises activated carbon.

8. Device (30) according to claim 7, wherein the activated carbon is granular.

9. Device (30) according to any one of the preceding claims, comprising a tap (42) for coupling with a conduction (33) for the inlet of water to the device (30), and/or a tap (42) for coupling with a conduction (33) for the outlet of water from the device (30).

10. Device (30) according to any one of the preceding claims, wherein the fluorescence measurement comprises at least one of the following parameters:
- basal fluorescence;
- photosynthetic efficiency or photosynthetic yield, YII;
- *yield of regulated non-photochemical fluorescence quenching,* Y(NPQ);
- yield of non-regulated non-photochemical *fluorescence* quenching, YNO);
- or a combination of the above.

11. Device (30) according to any one of the preceding claims, wherein the purifying filter (8) is installed within a purifying chamber (12) between the first chamber (2) and the second chamber (1).

12. Device (30) according to any one of the preceding claims, further comprising a data communication unit (23) configured to collect and transmit the measurements acquired by the data acquisition unit (18).

13. Device (30) according to claim 12, wherein the communication unit (23) is wireless.

14. System for monitoring toxic substances in water, **characterized in that** it comprises:
one or more devices (30) according to one of the preceding claims 1 to 13;
a surface equipment (19) configured to house outside the water a communication unit (23) and a processor (24), wherein the surface equipment (19) further comprises a battery (27) and a photovoltaic solar panel (28) for generating electrical energy, wherein the battery (27) stores the generated electrical energy and feeds at least the processor (24) and the communication unit (23).

15. System for monitoring toxic substances according to claim 14, further comprising a remote computer (21) and a cloud server (20) configured to communicate with the surface equipment (19), where the remote computer (21) is configured to compare the measurements acquired in both chambers (1,2) of the device (30), where the remote computer (21) is further configured to send an alarm to one or more surveillance terminals (22) based on the result of the comparison.

16. System for monitoring toxic substances according to claim 14 or 15, further comprising a remote computer (21) configured to communicate with the device (30) according to claim 13.

## Patentansprüche

1. Vorrichtung (30) zum Überwachen von toxischen Substanzen im Wasser, **dadurch gekennzeichnet, dass** sie Folgendes umfasst:
- eine erste Überwachungskammer (1) mit einem Einlass und einem Auslass für den Wasserfluss, umfassend ein Gehäusemodul (10), das dazu konfiguriert ist, mindestens einen Biofilm (5) und ein Fluorometer (6) aufzunehmen, wobei das Fluorometer dazu konfiguriert ist, die Fluoreszenz des Biofilms (5) zu messen;
- eine zweite Referenzkammer (2) mit einem Einlass und einem Auslass für den Wasserfluss, umfassend ein Gehäusemodul (10), das dazu konfiguriert ist, mindestens einen Biofilm (5) und ein Fluorometer (6) aufzunehmen, wobei das Fluorometer dazu konfiguriert ist, die Fluoreszenz des Biofilms (5) zu messen;
- einen Reinigungsfilter (8), der sich zwischen der ersten Kammer (1) und der zweiten Kammer (2) befindet, sodass derselbe Wasserfluss durch die erste Kammer (1) und die zweite Kammer (2) fließt, nachdem er den Reinigungsfilter (8) durchlaufen hat;
wobei beide Kammern (1, 2) dazu konfiguriert sind, während der Verwendung zu ermöglichen, dass der Biofilm (5) Außenlicht empfängt;
wobei die Vorrichtung (30) ferner eine Datenerfassungseinheit (18) umfasst, die dazu konfiguriert ist, die Fluoreszenzmessung vom Fluorometer (6) jeder Kammer (1, 2) zu erfassen.

2. Vorrichtung (30) nach Anspruch 1, ferner umfassend einen passiven Probenehmer für organische Schadstoffe (3).

3. Vorrichtung (30) nach Anspruch 1 oder 2, ferner umfassend einen passiven Probenehmer für anorganische Schadstoffe (4).

4. Vorrichtung (30) nach einem der Ansprüche 1 bis 3, ferner umfassend einen Phytoplanktonauffangfilter (7).

5. Vorrichtung (30) nach einem der vorhergehenden Ansprüche, umfassend ein Filtergewebe (11) am Einlass und Auslass der Kammer (1, 2) für den Wasserfluss, wobei das Filtergewebe (11) inert ist.

6. Vorrichtung (30) nach Anspruch 5, wobei das Filtergewebe (11) 316-Edelstahl ist.

7. Vorrichtung (30) nach einem der vorhergehenden Ansprüche, wobei der Reinigungsfilter (8) Aktivkohle umfasst.

8. Vorrichtung (30) nach Anspruch 7, wobei die Aktivkohle körnig ist.

9. Vorrichtung (30) nach einem der vorhergehenden Ansprüche, umfassend einen Hahn (42) zum Koppeln mit einer Leitung (33) für den Einlass von Wasser in die Vorrichtung (30) und/oder einen Hahn (42) zum Koppeln mit einer Leitung (33) für den Auslass von Wasser aus der Vorrichtung (30).

10. Vorrichtung (30) nach einem der vorhergehenden Ansprüche, wobei die Fluoreszenzmessung mindestens einen der folgenden Parameter umfasst:
- Basalfluoreszenz;
- photosynthetische Effizienz oder photosynthetische Ausbeute, YII;
- *Ausbeute der regulierten nicht-photochemischen Fluoreszenz-Löschung*, Y(NPQ);
- Ausbeute der unregulierten nicht-photochemischen Fluoreszenz-Löschung, YNO);
- oder eine Kombination der vorgenannten.

11. Vorrichtung (30) nach einem der vorhergehenden Ansprüche, wobei der Reinigungsfilter (8) innerhalb einer Reinigungskammer (12) zwischen der ersten Kammer (2) und der zweiten Kammer (1) installiert ist.

12. Vorrichtung (30) nach einem der vorhergehenden Ansprüche, ferner umfassend eine Datenkommunikationseinheit (23), die dazu konfiguriert ist, die von der Datenerfassungseinheit (18) erfassten Messungen zu sammeln und zu übertragen.

13. Vorrichtung (30) nach Anspruch 12, wobei die Kommunikationseinheit (23) drahtlos ist.

14. System zum Überwachen von toxischen Substanzen im Wasser, **dadurch gekennzeichnet, dass** es Folgendes umfasst:
ein oder mehrere Vorrichtungen (30) nach einem der vorhergehenden Ansprüche 1 bis 13;
eine Oberflächeneinrichtung (19), die dazu konfiguriert ist, außerhalb des Wassers eine Kommunikationseinheit (23) und einen Prozessor (24) aufzunehmen, wobei die Oberflächeneinrichtung (19) ferner eine Batterie (27) und ein photovoltaisches Sonnenpaneel (28) zum Generieren von elektrischer Energie umfasst, wobei die Batterie (27) die generierte elektrische Energie speichert und mindestens den Prozessor (24) und die Kommunikationseinheit (23) versorgt.

15. System zum Überwachen von toxischen Substanzen nach Anspruch 14, ferner umfassend einen entfernten Computer (21) und einen Cloud-Server (20), die dazu konfiguriert sind, mit der Oberflächeneinrichtung (19) zu kommunizieren, wobei der entfernte Computer (21) dazu konfiguriert ist, die in beiden Kammern (1, 2) der Vorrichtung (30) erfassten Messungen zu vergleichen, und wobei der entfernte Computer (21) ferner dazu konfiguriert ist, basierend auf dem Vergleichsergebnis einen Alarm an ein oder mehrere Überwachungsterminals (22) zu senden.

16. System zum Überwachen von toxischen Substanzen nach Anspruch 14 oder 15, ferner umfassend einen entfernten Computer (21), der dazu konfiguriert ist, mit der Vorrichtung (30) nach Anspruch 13 zu kommunizieren.

## Revendications

1. Dispositif (30) de surveillance de substances toxiques dans l'eau, **caractérisé en ce qu'**il comprend :
- une première chambre de surveillance (1) comportant une entrée et une sortie pour le flux d'eau, comprenant un module de logement (10) configuré pour loger au moins un biofilm (5) et un fluorimètre (6), dans laquelle le fluorimètre est configuré pour mesurer la fluorescence du biofilm (5) ;
- une seconde chambre de référence (2) comportant une entrée et une sortie pour le flux d'eau, comprenant un module de logement (10) configuré pour loger au moins un biofilm (5) et un fluorimètre (6), dans laquelle le fluorimètre est configuré pour mesurer la fluorescence du biofilm (5) ;
- un filtre de purification (8) situé entre la première chambre (1) et la seconde chambre (2), de telle sorte qu'un même flux d'eau traverse la première chambre (1), puis le filtre de purification (8) et la seconde chambre (2) ;
dans lequel les deux chambres (1, 2) sont configurées pour permettre, lors de l'utilisation, au biofilm (5) de recevoir de la lumière extérieure ;
dans lequel le dispositif (30) comprend en outre une unité d'acquisition de données (18) configurée pour acquérir la mesure de fluorescence provenant du fluorimètre (6) de chaque chambre (1, 2).

2. Dispositif (30) selon la revendication 1, comprenant en outre un échantillonneur passif de polluants organiques (3).

3. Dispositif (30) selon la revendication 1 ou 2, comprenant en outre un échantillonneur passif de polluants inorganiques (4).

4. Dispositif (30) selon l'une quelconque des revendications 1 à 3, comprenant en outre un filtre de capture du phytoplancton (7).

5. Dispositif (30) selon l'une quelconque des revendications précédentes, comprenant une toile filtrante (11) au niveau de l'entrée et de la sortie de la chambre (1, 2) pour le flux d'eau, dans lequel la toile filtrante (11) est inerte.

6. Dispositif (30) selon la revendication 5, dans lequel la toile filtrante (11) est en acier inoxydable 316.

7. Dispositif (30) selon l'une quelconque des revendications précédentes, dans lequel le filtre de purification (8) comprend du charbon actif.

8. Dispositif (30) selon la revendication 7, dans lequel le charbon actif est granulaire.

9. Dispositif (30) selon l'une quelconque des revendications précédentes, comprenant un robinet (42) destiné à être raccordé à une conduite (33) pour l'entrée d'eau dans le dispositif (30), et/ou un robinet (42) destiné à être raccordé à une conduite (33) pour la sortie d'eau du dispositif (30).

10. Dispositif (30) selon l'une quelconque des revendications précédentes, dans lequel la mesure de fluorescence comprend au moins l'un des paramètres suivants :
- la fluorescence basale ;
- l'efficacité photosynthétique ou le rendement photosynthétique, YII ;
- le rendement de l'extinction non photochimique régulée de la fluorescence, Y(NPQ) ;
- le rendement de l'extinction non photochimique non régulée de la fluorescence, Y(NO) ;
- ou une combinaison de ceux-ci.

11. Dispositif (30) selon l'une quelconque des revendications précédentes, dans lequel le filtre de purification (8) est installé à l'intérieur d'une chambre de purification (12) entre la première chambre (2) et la seconde chambre (1).

12. Dispositif (30) selon l'une quelconque des revendications précédentes, comprenant en outre une unité de communication de données (23) configurée pour collecter et transmettre les mesures acquises par l'unité d'acquisition de données (18).

13. Dispositif (30) selon la revendication 12, dans lequel l'unité de communication (23) est sans fil.

14. Système de surveillance de substances toxiques dans l'eau, **caractérisé en ce qu'**il comprend :
un ou plusieurs dispositifs (30) selon l'une quelconque des revendications précédentes 1 à 13 ;
un équipement de surface (19) configuré pour loger, hors de l'eau, une unité de communication (23) et un processeur (24), dans lequel l'équipement de surface (19) comprend en outre une batterie (27) et un panneau solaire photovoltaïque (28) destiné à produire de l'énergie électrique, dans lequel la batterie (27) stocke l'énergie électrique produite et alimente au moins le processeur (24) et l'unité de communication (23).

15. Système de surveillance de substances toxiques selon la revendication 14, comprenant en outre un ordinateur distant (21) et un serveur en nuage (20) configurés pour communiquer avec l'équipement de surface (19), dans lequel l'ordinateur distant (21) est configuré pour comparer les mesures acquises dans les deux chambres (1, 2) du dispositif (30), dans lequel l'ordinateur distant (21) est en outre configuré pour envoyer une alarme à un ou plusieurs terminaux de surveillance sur la base du résultat de la comparaison.

16. Système de surveillance de substances toxiques selon la revendication 14 ou 15, comprenant en outre un ordinateur distant (21) configuré pour communiquer avec le dispositif (30) selon la revendication 13.
